# EUROPEAN PATENT SPECIFICATION

(11) **EP 1 838 287 B1**
(45) Date of publication and mention of the grant of the patent: **23.05.2012**
(21) Application number: 06706167.1
(22) Date of filing: 05.01.2006
(51) Int. Cl.: A61K 9/16, A61K 31/43, A61P 31/04

(54) **PROCESS FOR PREPARING GRANULATES COMPRISING AMOXICILLIN**
VERFAHREN ZUR GRANULATHERSTELLUNG MIT AMOXICILLIN
PROCEDE DE PREPARATION DE GRANULES COMPRENANT DE L' AMOXICILLINE

(30) Priority: 07.01.2005 SI 200500005
(43) Date of publication of application: 03.10.2007
(73) Proprietor: SANDOZ AG, 4056 Basel (CH)
(72) Inventor: SCHWARZ, Franz, Xaver, A-6300 Wörgl (AT)
(74) Representative: Wichmann, Hendrik
(86) International application number: PCT/EP2006/000053
(87) International publication number: WO 2006/072577

(56) References cited:
- WO-A-95/25516
- WO-A-99/62910
- FR-A- 2 445 833
- GB-A- 1 576 731
- US-A- 4 607 029
- US-A- 6 077 536
- US-A1- 2003 224 049

## Description

### Field of the Invention

The present invention belongs to the field of pharmaceutical industry and relates to a novel process for preparing a stable granulate, comprising a mixture of amoxicillin trihydrate and amoxicillin sodium, by means of extrusion granulation of amoxicillin trihydrate with an aqueous solution of sodium hydroxide, sodium bicarbonate, sodium carbonate and mixture thereof or with an aqueous solution of a sodium salt of (C₁₋₈) organic acid as a sodium source, and to a method for the treatment of bacterial infection in humans or animals. Alternatively, the present invention relates to a novel process for preparing a stable granulate comprising pure amoxicillin sodium as defined in the claims, by means of said extrusion granulation of amoxicillin trihydrate.

In a further aspect the present invention relates to the novel stable granulate comprising the mixture of amoxicillin trihydrate and amoxicillin sodium.

### Technical problem

There is a constant need for preparing stable dosage forms of a combination of amoxicillin trihydrate and amoxicillin sodium or alternatively, pure amoxicillin sodium, e.g. granulate or tablets, for use in humans and animals, which may be used for reconstitution of said granulate with water into aqueous suspension, for parenteral injection administration, for preparing solid dosage forms, e.g. tablets, dispersible tablets, capsules, powders, chewable tablets.

### Prior art

Amoxicillin is an antibacterial agent extensively used for the treatment of a wide range of bacterial infections, e.g. for the treatment of respiratory infections and urinary tract infections. Amoxicillin is described in The Merck Index, An Enzydopedia of Chemicals, Drugs, and Biologicals, 13Ed, 2001, under monographic number 582. Amoxicillin trihydrate is the stable form of amoxicillin and is available on the market in a number of different formulations for oral administration, for example, as granulate or powders, oral suspension, hard gelatine capsules, tablets, chewable tablets, pediatric drops. Amoxicillin sodium is used in the therapy for parenteral administration. Amoxicillin in the form of hydrates and alkali salts was described for the first time in the British patent no. 1,241,844.

Patent literature describe several processes for the preparation of amoxicillin sodium salt. For example, in one of them amoxicillin trihydrate is suspending in methylene dichloride, treating with suitable amine, e.g. diethylamine or triethylamine as solubilizing agent to form amine salt, adding methanol to facilitate dissolution, dehydrating with "molecular sieve, filtering and finally precipitating the sodium salt of amoxicillin by the addition of sodium 2-ethylhexanoate to the filtrate. On commercial scale the product is found to be contaminated by residual reagents such as triethylamine or excessive amounts of residual solvents. Drying from aqueous systems is difficult due to very high aqueous solubility of amoxicillin sodium. Furthermore, the low stability of aqueous solution of amoxicillin sodium, enhanced by its reletively high pH value, makes drying more difficult.

British patent no. 1,527,557 describes a process for the preparation of solid amoxicillin sodium by freeze-drying a solution of amoxicillin sodium in a solvent system.

British patents no. 1,578,731 and no. 2,096,599 describe a process for the preparation of solid amoxicillin sodium by spray-drying a solution of amoxicillin sodium in aqueous isopropyl alcohol or aqueous tert-butanol. When amoxicillin sodium is prepared by spray-drying or freeze-drying of an aqueous solution of amoxicillin sodium the content of degradation products, e.g. polymeric products and water content may be extremely high, which negatively influence to the stability of the product.

Patent EP-B-0 131 147 describes crystalline amoxicillin sodium and the process for its preparation. The process use large amounts of solvent mixture, e.g. methanol, methyl acetate and methylene chloride and amine, e.g. triethylamine, is used as solubilizing agent for starting amoxicillin trihydrate.

EP-B-0 596 262 describes a process for preparing sterile amoxicillin sodium from amoxicillin trihydrate in an alcohol containing solvent, ih the presence of an amine, e.g. triethylamine, and sodium carboxylate, but the solvent mixture used is even more complex than that described in EP-B-0 131 147 and besides metanol and methyl acetate a further C₂₋₅ alcohol is used.

Presumably for above reason commercially available sodium amoxicillin has been obtained by spray-drying method. Amoxicillin sodium is highly water soluble antibiotic used for parenteral administration, but is significantly less stable than amoxicillin trihydrate, in particular on account of its pronounced hygroscopic nature.

All of above described processes require a high level of technical effort or cost expenditure and these disadvantages could be the reason that amoxicillin sodium is currently hardly available on the market for oral use.

Oral forms of amoxicillin sodium have been described in the patent literature, but such forms are currently not available on the market. WO 94/00112 describes an extended-release preparation containing amoxicillin sodium for oral administration for the treatment of infections caused by *Heliobacter pylori* in the upper gastrointestinal tract. WO 98/40054 describes an enteric coated oral dosage form comprising amoxicillin sodium for use in the treatment of *Heliobacter pylori* infections.

US patent no. 6,756,057 describes that bacterial infections may be treated using high dosage regimen of amoxicillin and potassium clavulanate. Preferably, the dosage is provided by a bilayered tablet. Formulations provide a unit dosage of amoxicillin and potassium clavulanate, wherein the first immediate release phase contain all of potassium clavulanate and from 0% to 50% of the amoxicillin in the forum of trihydrate and a slow release phase containing from 50% to 100% of amoxicillin in the form of crystallized sodium salt, and citric acid. Commercial drug Augmentin^{®} XR has been marketed in the USA and also in Europe on the basis of said patent.

US 6,077,536 discloses a process for preparing granulates for oral administration comprising amoxicillin and a sodium source. However, the amoxicillin is still present as trihydrate during storage; the sodium salt is only formed in situ upon reconstitution in 5 water prior to administration.

WO 95/25516 discloses a process for preparing granules comprising amoxicillin trihydrate.

### Description of the invention

The object of the present invention is to find a novel process for preparing a stable granulate comprising a mixture of amoxicillin trihydrate and amoxicillin sodium or alternatively, to find a novel process for preparing a stable granulate comprising pure amoxicillin sodium, wherein the exacting spray-drying or freeze-drying metods and the use of organic solvents and amine, e.g. triethylamine, used in the prior art processes for preparing amoxicillin sodium, would be omitted. The ratio of amoxicillin trihydrate to amoxicillin sodium to one another in the granulate can be adjusted with the quantity of the sodium salt source to starting amoxicillin trihydrate used according to the novel process of the invention.

In a further aspect the present invention relates to the novel stable granulate comprising the mixture of amoxicillin trihydrate and amoxicillin sodium whenever prepared according to the above described process.

We have surprisingly and unexpectedly found that the problem known in the prior art has been solved by the novel process of the present invention, wherein amoxicillin trihydrate is extruded with an aqueous solution of sodium hydroxide, sodium bicarbonate, sodium carbonate and mixture thereof or with an aqueous solution of a sodium salt of (C₁₋₈) organic acid as a sodium source, by means of commercially available extruder to obtain granulate of the mixture of amoxicillin trihydrate and amoxicillin sodium. Amoxicillin sodium in said mixture with amoxicillin trihydrate may thus be used for oral administration.

The ratio of above sodium source to starting amoxicillin trihydrate used according to the process of the present invention for preparing said antibiotic mixture in the granulate can vary from about 0.20 to about 0.95 mol of sodium source to 1 mol of amoxicillin trihydrate.

A further aspect of the present invention provides a novel process for preparing granulate comprising pure amoxicillin sodium as defined in the claims wherein amoxicillin trihydrate is extruded with the aqueous solution of sodium hydroxide, sodium bicarbonate, sodium carbonate and mixture thereof or with the aqueous solution of the sodium salt of (C₁₋₈) organic acid as the sodium source by means of commercially available extruder.

The ratio of above sodium source to starting amoxicillin trihydrate used according to the process of the invention for preparing granulate comprising pure amoxicillin sodium in the granulate is suitable in approximately equivalent amounts.

The term "pure" amoxicillin sodium means amoxicillin sodium free of any residual organic solvent, e.g. methanol or methylene chloride, and free of any traces of amines, e.g. triethylamine.

The yields of extrusion granulation according to the present invention are high.

Extrusion process and extruders are known in the art, for example as described in J. Swarbrich and James C. Boylan, Encyclopedia of Pharmaceutical Technology, Vol. 5, 1992, pp 395-442.

EP-B- 0 080 862 relates to water-dispersible composition for oral administration in the treatment of bacterial infections comprising amoxicillin trihydrate and potassium clavulanate and conventional excipients, wherein it contains a non-hygroscopic water-soluble binder, e.g. hydroxypropylmethylcellulose, in an amount sufficient to render said composition extrudable after addition of an organic solvent for a binder.

According to the process of the present invention the extrusion granulation of amoxicillin trihydrate with the aqueous solution of sodium hydroxide, sodium bicarbonate, sodium carbonate and mixture thereof or with an aqueous solution of sodium salt of (C₁₋₈) organic acid as the sodium source is performed at appropriate extrusion temperatures, e.g. between 0°C to 60°C, preferably between 10°C to 40°C to obtain moist extruded mass. The obtained moist extruded mass is granulated through a sieve, subsequently dried the obtained granulate, which is optionally re-dryed to obtain the dry granulate of the mixture of amoxicillin trihydrate and amoxicillin sodium in the form of a mixture of crystal form, amorphous form and agglomerates. Said antibiotic ixture form has not been disclosed in the prior art and is therefore novel.

Alternatively, extrusion granulation of amoxicillin trihydrate suitably in approximately equivalent amount to sodium source according to above described process provides granulate comprising pure amoxicillin sodium.

As the sodium salt of (C₁₋₈) organic acid for extrusion granulation process may be used the sodium salt of acetic acid, propionic acid, butyric acid, valeric acid, caproic acid, caprilic acid and capric acid, preferably sodium salt of 2-ethyl hexanoate (sodium salt of 2-ethyl caproic acid).

Mesh size of the sieve used in the extrusion granulation process is in the range between 0.5 mm to 4.0 mm, preferably in the range from 1 mm to 2 mm, most preferably 1.25 mm.

Granulate comprising the mixture of amoxicillin trihydrate and amoxicillin sodium may be prepared according to the process of the present invention, comprising the following steps:
a. extruding amoxicillin trihydrate with an aqueous solution of sodium hydroxide, sodium bicarbonate, sodium carbonate and mixture thereof or with an aqueous solution of sodium salt of (C₁₋₈) organic acid as a sodium source to form a moist extruded mass,
b. granulating moist extruded mass through a sieve,
c. drying the obtained granulate, and
d. optionally re-drying granulate to obtain the dry granulate of the mixture of amoxicillin trihydrate and amoxicillin sodium

Granulate comprising pure amoxicillin sodium may be prepared according to the above steps but using in step a. amoxicillin trihydrate and sodium salt source suitable in approximately equivalent amounts.

Granulate comprising the mixture of amoxicillin trihydrate and amoxicillin sodium or alternatively, granulate comprising pure amoxicillin sodium obtained according to the process of the present invention is stable and may be presented in protective packages such as glass bottles, aluminium foil sachets containing multiple doses, or in single dose sachets, or into other suitable container protected from moisture. Granulate according to the present invention is free of any residual organic solvent, e.g. methanol or methylene chloride, and free of residual reagents such as triethylamine or other amines which were used in the prior art for preparing amoxicillin sodium.

A preferred mean particle size of the granules is in the range between 200 µm to 3000 µm, particularly between 800 µm to 1500 µm.

The dried extrudate (granulate) may be levelled as required through a more or less coarse sieve or ground with a mill. In this way powders and granulate comprising the mixture of amoxicillin trihydrate and amoxicillin sodium or alternatively, granulate and powders comprising pure amoxicillin sodium with different particle distribution can be produced.

Granulate of the present invention can also be produced discontinuously by granulation in a granulator, e.g. ribbon blenders Stephan, Disona, Collte Gral, or in a fluidised bed granulator by spray granulation, e.g. Glatt Hüttlin.

Granulate according to the invention may be directly tabletted after the addition of conventional tabletting adjuvants, e.g. flow-regulating agents, lubricants and tabletting auxiliaries.

Granular product obtained according to the present invention may be used at a range of unit doses, for example between 1 g to 5 g of the mixture of amoxicillin trihydrate and amoxicillin sodium. Granulate comprising pure amoxicillin sodium may be used for oral administration or for parenteral administration.

The invention also provides granulate comprising the mixture of amoxicillin trihydrate and amoxicillin sodium, for reconstitution with water into an aqueous suspension, for use in the treatment of bacterial infections.

The invention also provides a method of treatment of bacterial infections in humans and animals which comprises the administration of a therapeutically effective amount of the mixture of amoxicillin trihydrate and amoxicillin sodium containing in the granulate.

The invention is illustrated but in no way limited by the following Examples:

All examples use extrusion granulation technique on the following equipment under the conditions stated, moist granulated through a 2 mm sieve and dried in the specific fluidised bed equipment. Optionally, re-drying can take place by introducing absolutely dry air through the granulate bed at room temperature.

Extruder: Theysohn TSK N20/20D - Maschinenbau / Salzgitter (Germany)

| | |
|---|---|
| Addition of amoxycillin trihydrate powder | 80-125 g/min |
| Extruding liquid rate | 15-30 g/min |

Drying:

| | |
|---|---|
| Fluidised bed dryer Glatt GPC2 | |
| Incoming temperature | 85°C |
| Material temperature at the end of drying | 45-55°C |

Re-drying (optional):

| | |
|---|---|
| Drying air | < 5% relative air humidity at 25°C |
| Duration | 12-72 h |

### Example 1:

Granulate comprising pure amoxicillin sodium

| Component | weight (g) |
|---|---|
| Amoxicillin trihydrate | 1000.0 |

Solution for extrusion granulation:

| Component | weight (g) |
|---|---|
| NaOH | 95.4 |
| water | 238.4 |

Amoxicillin trihydrate is extruded with the aqueous solution of sodium hydroxide under above described conditions, sieved and dried to obtain 923.6 g granulate comprising pure amoxicillin sodium.

### Example 2:

Granulate comprising the mixture of amoxicillin trihydrate and amoxicillin sodium

| Component | weight (g) |
|---|---|
| Amoxicillin trihydrate | 1000.0 |

Solution for extrusion granulation:

| Component | weight (g) |
|---|---|
| NaOH | 47.7 |
| water | 220.0 |

Amoxicillin trihydrate is extruded with the aqueous solution of sodium hydroxide under above described conditions, sieved and dried to obtain granulate comprising the mixture of 500.0 g amoxicillin trihydrate and 461.8 g of amoxicillin sodium.

### Example 3:

Granulate comprising the mixture of amoxicillin trihydrate and amoxicillin sodium

| Component | weight (g) |
|---|---|
| Amoxicillin trihydrate | 1000.0 |

Solution for extrusion granulation:

| Component | weight (g) |
|---|---|
| Na₂CO₃ | 63.2 |
| water | 230.0 |

Amoxicillin trihydrate is extruded with the aqueous solution of sodium carbonate under above described conditions, sieved and dried to obtain granulate comprising the mixture of 500.0 g of amoxicillin trihydrate and 461.8 g of amoxicillin sodium.

### Example 4

Granulate comprising pure amoxicillin sodium

| Component | weight (g) |
|---|---|
| Amoxicillin trihydrate | 1000.0 |
| Na₂CO₃ | 126.3 |

A mixture of amoxicillin trihydrate and sodium carbonate is extruded with 220.0 g of water under above described conditions, sieved and dried to obtain 923.6 g of granulate comprising pure amoxicillin sodium.

### Example 5

Granulate comprising the mixture of amoxicillin trihydrate and sodium amoxicillin

| Component | weight (g) |
|---|---|
| Amoxicillin trihydrate | 1000.0 |
| NaHCO₃ | 70.1 |
| water | 220.0 |

A mixture of 1000.0 g amoxicillin trihydrate, 70.1 g of sodium bicarbonate and 220.0 g of water is granulated for 3 minutes in the Stephan mixer, then sieved and dried according to above conditions to obtain granulate comprising the mixture of 650.0 g amoxicillin trihydrate and 323.2 g of amoxicillin sodium.

## Claims

1. A process for preparing a stable granulate of a mixture comprising amoxicillin trihydrate and amoxicillin sodium, the process comprising the following steps:
a. extruding amoxicillin trihydrate with an aqueous solution of sodium hydroxide, sodium bicarbonate, sodium carbonate and mixture thereof or with an aqueous solution of the sodium salt of (C₁₋₈) organic acid as a sodium source to form a moist extruded mass,
b. granulating moist extruded mass through a sieve,
c. drying the obtained granulate, and
d. optionally re-drying granulate to obtain the dry granulate of the mixture of amoxicillin trihydrate and amoxicillin sodium.

2. The process according to claim 1a, wherein (C₁₋₈) organic acid is selected from the group consisting of acetic acid, propionic acid, butyric acid, valeric acid, caproic acid, caprylic acid and capric acid.

3. The process according to claim 1a and 2, wherein the sodium salt of (C₁₋₈) organic acid is sodium 2-ethyl hexanoate.

4. The process according to claim 1a, wherein the ratio of sodium source to amoxicillin trihydrate is between about 0.20 mol to about 0.95 mol of sodium source to 1 mol of amoxicillin trihydrate.

5. The process according to claim 1a, wherein the extrusion temperature is between 0°C to 60°C.

6. The process according to claim 1a, wherein the extrusion temperature is between 10°C to 40°C.

7. The process according to any one of claims 1 to 6, wherein the particle size of the granulate is in the range of 200 µm to 3000 µm.

8. The process according to any one of claims 1 to 7, wherein the particle size of the granulate is in the range of 800 µm to 1500 µm.

9. A granulate prepared according to claim 1 comprising a mixture of amoxicillin trihydrate and amoxicillin sodium in the form of a mixture of crystal form, amorphous form and agglomerates.

10. The granulate according to claim 9, free of any residual organic solvent.

11. The granulate according to claim 9 for reconstitution into an aqueous suspension prior to use.

12. The granulate according to claim 9, which is provided in glass bottles, unit dose sachets or into other suitable container protected from moisture.

13. A therapeutically effective amount of the mixture of amoxicillin trihydrate and amoxicillin sodium contained in the granulate prepared according to any of claims 1-8, for use in a method of treatment of bacterial infections in humans or animals, which comprises the administration of said amount to said human or animal.

14. Use of the granulate according to claim 9 in the manufacture of a medicament for treating bacterial infections.

15. A process for preparing a stable granulate comprising amoxicillin sodium free of any residual organic solvent and free of any traces of amine, the process comprising the following steps:
a. extruding amoxicillin trihydrate with an aqueous solution of sodium hydroxide, sodium bicarbonate, sodium carbonate and mixture thereof or with an aqueous solution of the sodium salt of (C₁₋₈) organic acid as a sodium source suitably in approximately equivalent amounts to form a moist extruded mass,
b. granulating moist extruded mass through a sieve,
c. drying the obtained granulate, and
d. optionally re-drying granulate to obtain the dry granulate of pure amoxicillin sodium.

## Patentansprüche

1. Verfahren zum Herstellen eines stabilen Granulats einer Mischung, umfassend Amoxicillintrihydrat und Amoxicillinnatrium, wobei das Verfahren die folgenden Schritte umfasst:
a. Extrudieren von Amoxicillintrihydrat mit einer wässerigen Lösung von Natriumhydroxid, Natriumbicarbonat, Natriumcarbonat und einer Mischung davon oder mit einer wässerigen Lösung des Natriumsalzes von (C₁₋₈) organischer Säure als eine Natriumquelle unter Bildung einer feuchten extrudierten Masse,
b. Granulieren der feuchten extrudierten Masse durch ein Sieb,
c. Trocknen des erhaltenen Granulats, und
d. gegebenenfalls erneutes Trocknen des Granulats, unter Bildung des Trockengranulats der Mischung von Amoxicillintrihydrat und Amoxicillinnatrium.

2. Verfahren nach Anspruch 1a, wobei (C₁₋₈) organische Säure ausgewählt ist aus der Gruppe, bestehend aus Essigsäure, Propionsäure, Buttersäure, Valeriansäure, Capronsäure, Caprylsäure und Caprinsäure.

3. Verfahren nach Anspruch 1a und 2, wobei das Natriumsalz der (C₁₋₈) organischen Säure Natrium-2-ethylhexanoat ist.

4. Verfahren nach Anspruch 1a, wobei das Verhältnis der Natriumquelle zu Amoxicillintrihydrat zwischen etwa 0,20 Mol bis etwa 0,95 Mol Natriumquelle zu 1 Mol Amoxicillintrihydrat beträgt.

5. Verfahren nach Anspruch 1a, wobei die Extrusionstemperatur zwischen 0°C bis 60°C beträgt.

6. Verfahren nach Anspruch 1a, wobei die Extrusionstemperatur zwischen 10°C bis 40°C beträgt.

7. Verfahren nach einem beliebigen der Ansprüche 1 bis 6, wobei die Partikelgröße des Granulats in dem Bereich von 200 µm bis 3.000 µm liegt.

8. Verfahren nach einem beliebigen der Ansprüche 1 bis 7, wobei die Partikelgröße des Granulats in dem Bereich von 800 µm bis 1.500 µm liegt.

9. Granulat, hergestellt gemäß Anspruch 1, umfassend eine Mischung von Amoxicillintrihydrat und Amoxicillinnatrium in der Form einer Mischung von kristalliner Form, amorpher Form und Agglomeraten.

10. Granulat nach Anspruch 9, frei von jeglichem rückständigem organischem Lösungsmittel.

11. Granulat nach Anspruch 9 zur Rekonstitution in eine wässerige Suspension vor der Verwendung.

12. Granulat nach Anspruch 9, das in Glasflaschen, Einheitsdosierungspäckchen oder in anderen geeigneten Behältern, geschützt vor Feuchtigkeit, bereitgestellt ist.

13. Therapeutisch wirksame Menge der Mischung von Amoxicillintrihydrat und Amoxicillinnatrium, enthalten in dem gemäß einem beliebigen der Ansprüche 1-8 hergestellten Granulat, zur Verwendung in einem Verfahren zur Behandlung von bakteriellen Infektionen in Menschen oder Tieren, das die Verabreichung der Menge an den Menschen oder das Tier umfasst.

14. Verwendung des Granulats nach Anspruch 9 in der Herstellung eines Medikaments zur Behandlung von bakteriellen Infektionen.

15. Verfahren zum Herstellen eines stabilen Granulats, umfassend Amoxicillinnatrium, frei von jeglichem rückständigem organischem Lösungsmittel und frei von jeglichen Spuren von Amin, wobei das Verfahren die folgenden Schritte umfasst:
a. Extrudieren von Amoxicillintrihydrat mit einer wässerigen Lösung von Natriumhydroxid, Natriumbicarbonat, Natriumcarbonat und einer Mischung davon oder mit einer wässerigen Lösung des Natriumsalzes von (C₁₋₈) organischer Säure als eine Natriumquelle, geeigneter Weise in ungefähr äquivalenten Mengen, unter Bildung einer feuchten extrudierten Masse,
b. Granulieren der feuchten extrudierten Masse durch ein Sieb,
c. Trocknen des erhaltenen Granulats, und
d. gegebenenfalls erneutes Trocknen des Granulats, unter Bildung des Trockengranulats von reinem Amoxicillinnatrium.

## Revendications

1. Procédé de préparation d'un granulé stable d'un mélange comprenant de l'amoxicilline trihydrate et de l'amoxicilline sodique, le procédé comprenant les étapes suivantes :
a. extrusion d'amoxicilline trihydrate avec une solution aqueuse d'hydroxyde de sodium, de bicarbonate de soude, de carbonate de sodium et d'un mélange de ceux-ci ou avec une solution aqueuse du sel sodique d'un acide organique en (C₁₋₈) comme source de sodium pour former une masse extrudée humide,
b. granulation humide de la masse extrudée à travers un tamis,
c. séchage du granulé obtenu, et
d. éventuellement nouveau séchage du granulé pour obtenir le granulé sec du mélange d'amoxicilline trihydrate et d'amoxicilline sodique.

2. Procédé selon la revendication 1, dans lequel l'acide organique en (C₁₋₈) est choisi dans le groupe constitué par l'acide acétique, l'acide propionique, l'acide butyrique, l'acide valérique, l'acide caproïque, l'acide caprylique et l'acide caprique.

3. Procédé selon les revendications 1 et 2, dans lequel le sel sodique de l'acide organique en (C₁₋₈) est le 2-éthyl hexanoate de sodium.

4. Procédé selon la revendication 1, dans lequel le rapport de la source de sodium à l'amoxicilline trihydrate est compris entre environ 0,20 mole et environ 0,95 mole de source de sodium pour 1 mole d'amoxicilline trihydrate.

5. Procédé selon la revendication 1, dans lequel la température d'extrusion est comprise entre 0°C et 60°c.

6. Procédé selon la revendication 1, dans lequel la température d'extrusion est comprise entre 10°C et 40°C.

7. Procédé selon l'une quelconque des revendications 1 à 6, dans lequel la taille des particules du granulé est dans la gamme de 200 µm à 3 000 µm.

8. Procédé selon l'une quelconque des revendications 1 à 7, dans lequel la taille des particules du granulé est dans la gamme de 800 µm à 1 500 µm.

9. Granulé préparé selon la revendication 1 comprenant un mélange d'amoxicilline trihydrate et d'amoxicilline sodique sous la forme d'un mélange de forme cristalline, de forme amorphe et d'agglomérats.

10. Granulé selon la revendication 9, exempt de tout solvant organique résiduel.

11. Granulé selon la revendication 9 pour reconstitution en une suspension aqueuse avant l'utilisation.

12. Granulé selon la revendication 9, qui est fourni dans des bouteilles en verre, des sachets mono-dose ou dans d'autres récipients appropriés protégés de l'humidité.

13. Quantité thérapeutiquement efficace du mélange d'amoxicilline trihydrate et d'amoxicilline sodique contenue dans le granulé préparé selon l'une quelconque des revendications 1 à 8, pour une utilisation dans un procédé de traitement des infections bactériennes chez les humains ou les animaux, qui comprend l'administration de ladite quantité audit humain ou animal.

14. Utilisation du granulé selon la revendication 9 dans la fabrication d'un médicament destiné à traiter les infections bactériennes.

15. Procédé de préparation d'un granulé stable comprenant de l'amoxicilline sodique exempt de tout solvant organique résiduel et exempt de toutes traces d'amine, le procédé comprenant les étapes suivantes :
a. extrusion d'amoxicilline trihydrate avec une solution aqueuse d'hydroxyde de sodium, de bicarbonate de soude, de carbonate de sodium et d'un mélange de ceux-ci ou avec une solution aqueuse du sel sodique d'un acide organique en (C₁₋₈) comme source de sodium de manière convenable en quantités approximativement équivalentes pour former une masse extrudée humide,
b. granulation humide de la masse extrudée à travers un tamis,
c. séchage du granulé obtenu, et
d. éventuellement nouveau séchage du granulé pour obtenir le granulé sec d'amoxicilline sodique pure.
